Europäisches Patentamt

European Patent Office

Office européen des brevets

(19)

(11) Veröffentlichungsnummer : **0 030 681**
**B1**

(12) **EUROPÄISCHE PATENTSCHRIFT**

(45) Veröffentlichungstag der Patentschrift :
**13.06.84**

(21) Anmeldenummer : **80107587.0**

(22) Anmeldetag : **04.12.80**

(51) Int. Cl.³ : **A 61 K 31/635**, A 61 K 9/00 //
(A61K31/635, 31/565)

(54) Spritzfertige injizierbare wäßrige Lösungen der Alkalisalze von Canrenoinsäure und Furosemid und Verfahren zu ihrer Herstellung.

(30) Priorität : **18.12.79 DE 2950832**

(43) Veröffentlichungstag der Anmeldung :
**24.06.81 Patentblatt 81/25**

(45) Bekanntmachung des Hinweises auf die Patenterteilung : **13.06.84 Patentblatt 84/24**

(84) Benannte Vertragsstaaten :
**AT BE CH DE FR GB IT LI NL SE**

(56) Entgegenhaltungen :
**DE-A- 2 415 258**
**DE-A- 2 423 550**
**DE-A- 2 556 001**
**DICTIONNAIRE VIDAL, 1979, O.V.P. PARIS (FR), 55e Auflage Seite 1886, "SOLUDACTONE"**
**CHEMICAL ABSTRACTS, Band 74, Nr. 11, 15. März 1971, Zusammenfassung 50237f, Seite 63, COLUMBUS, OHIO (US) JANOS P. RADO et al.: "High doses of spironolactone (Aldactone, SC-14266 Veropsirone) alone and in combination with triamterene and (or) diuretics in the treatment of refractory edema associated with secondary hyperaldosteronism " & Endokrinologie 1970, 57(1), 46-62**
**UNLISTED DRUGS, Band 32, Heft 4, April 1980, CHATHAM N.J. (US) "Osyrol-Lasix", Seite 57**

(73) Patentinhaber : **HOECHST AKTIENGESELLSCHAFT**
**Postfach 80 03 20**
**D-6230 Frankfurt am Main 80 (DE)**

(72) Erfinder : **Leeb, Richard, Dr.**
**Nonnbornstrasse 15**
**D-6233 Kelkheim (Taunus) (DE)**
Erfinder : **Helbig, Rainer Joachim**
**Coburger Weg 9**
**D-6230 Frankfurt am Main 80 (DE)**

**Beschreibung**

Die Erfindung betrifft spritzfertige injizierbare wäßrige Lösungen des Gemisches der Alkalisalze von Canrenoinsäure und Furosemid. Diese Lösungen enthalten keinen Pufferzusatz und sind sterilisierbar, stabil und lokal verträglich.

Canrenoinsäure (3-(3-Oxo-17β-hydroxy-4,6-androstadien-17α-yl)-propionsäure) bzw. deren Kaliumsalz (K-Canrenoat) sowie Furosemid (4-Chlor-N-(2-furyl-methyl)-5-sulfamoylanthranilsäure) haben in der Medizin bereits als Diuretika Anwendung gefunden. Auch ein Kombinationspräparat für Injektionszwecke enthaltend ein Alkali-Canrenoat/Diureticum-Gemisch ist bereits beschrieben worden. So wird in der DE-PS 25 56 001 u.a. eine spritzgertige alkalische Lösung, bestehend aus einem Alkali-Canrenoat/Diureticum-Gemisch, Wasser und einem physiologisch verträglichen alkalischen Puffer beansprucht, welche dadurch gekennzeichnet ist, daß sie einen pH-Bereich von 10,2-11,2 aufweist und die Kapazität des Puffers nicht mehr als 0,1 Val pro Liter Injektionslösung in diesem pH-Bereich beträgt. Entsprechend den Angaben in der Beschreibung muß eine stabile, bei der Injektion physiologisch verträgliche und bei 120 °C sterilisierbare Lösung die genannten Merkmale aufweisen. Wesentlich ist, daß die Lösung den erwähnten pH-Wert besitzt und neben den Wirkstoffen zusätzlich einen Puffer geringer Pufferkapazität enthält.

Entgegen der in der genannten Auslegeschrift getroffenen Feststellungen wurde überraschenderweise gefunden, daß der Zusatz eines Puffers zur Erreichung der genannten Ziele bei Lösungen, die ein Gemisch der Alkalisalze von Canrenoinsäure und Furosemid enthalten und einen pH-Wert von über 10 aufweisen, nicht erforderlich ist. Solche wäßrigen Lösungen, die nur noch die Wirkstoffe als Alkalisalze und gegebenenfalls geeignete Isotonie-Zusätze, wie Natriumchlorid aber keinen zusätzlichen Puffer enthalten, gewährleisten optimale physiologische Verträglichkeit, ohne hinsichtlich Stabilität und Sterilisierbarkeit Nachteile gegenüber den Lösungen gemäß der erwähnten Patentschrift aufzuweisen. Die erfindungsgemäßen Lösungen werden als Arzneimittel verwendet.

Die Erfindung betrifft daher spritzfertige injizierbare wäßrige Lösungen, mit einem pH-Wert zwischen 10,2 und 11,2, dadurch gekennzeichnet, daß sie ein Gemisch der Alkalisalze von Canrenoinsäure und Furosemid ohne Pufferzusatz enthalten.

Die erfindungsgemäßen Lösungen enthalten also keinen Pufferzusatz. Sie weisen insbesondere einen pH-Wert zwischen 10,6 und 11,0, vorzugsweise einen solchen von 10,8, auf.

Von den Alkalicanrenoaten ist das Kaliumcanrenoat bevorzugt. Furosemid liegt vorzugsweise als Natriumsalz vor. Das Verhältnis von Alkalicanrenoat zu Furosemid beträgt vorzugsweise 30 : 1 bis 5 : 1, insbesondere 10 : 1. Eine Dosierungseinheit (1 Ampulle) enthält vorzugsweise 100 bis 400 mg, insbesondere 200 mg Alkalicanrenoat. Die Alkalicanrenoatkonzentration der wäßrigen Lösung beträgt zweckmäßigerweise 4 % bis 0,5 %, vorzugsweise liegt sie bei 1 bis 2 %.

Die Erfindung betrifft ferner ein Verfahren zur Herstellung von spritzfertigen, injizierbaren wäßrigen Lösungen mit einem pH-Wert zwischen 10,2 und 11,2, das dadurch gekennzeichnet ist, daß man Canrenoinsäure oder Alkalicanrenoat mit Furosemid in Wasser suspendiert und die Suspension mit wäßriger Alkalilösung auf einen pH-Wert von 10,2 bis 11,2 einstellt, und so in eine klare Lösung überführt. Als Alkalilösung kommen insbesondere Alkalihydroxidlösungen in Betracht, wobei Natriumhydroxyd bevorzugt wird.

Falls bei dem Verfahren die freie Canrenoinsäure eingesetzt und mit Alkalilösung der gewünschte pH-Wert eingestellt wird, so liegen beide Wirkstoffe als Salze mit dem gleichen Kation vor.

Es ist zweckmäßig, wenn während der Herstellung mit Inertgas, wie z. B. Stickstoff, begast wird.

Die erfindungsgemäßen Lösungen sind stabil, können bei 120 °C sterilisiert werden, sind physiologisch gut verträglich und können unverdünnt i. v. injiziert werden. Die Verabreichung als Kurzinfusion, gegebenenfalls unter Zusatz Glukose-Infusionslösung oder Isotonie-Zusätzen, wie Natriumchlorid, ist ebenfalls möglich. Die nachfolgenden Beispiele sollen die Erfindung erläutern.

**Beispiel 1**

800 ml Wasser für Injektionszwecke werden vorgelegt. Während der folgenden Herstellungsschritte wird mit Inertgas, z. B. Stickstoff, begast. In dem Wasser werden 10 g Kaliumcanrenoat gelöst und 1 g Furosemid suspendiert. Durch Zugabe von Natriumhydroxidlösung unter Rühren wird das Furosemid gelöst und das pH der Lösung auf 10,8 eingestellt. Mit Wasser für Injektionszwecke wird auf 1 l aufgefüllt. Die Lösung wird entkeimend filtriert, in Ampullen zu 20 ml abgefüllt und bei 120 °C 20 Minuten sterilisiert.

**Beispiel 2**

800 ml Wasser für Injektionszwecke werden vorgelegt. Während der folgenden Herstellungsschritte wird mit Inertgas, z. B. Stickstoff, begast. In dem Wasser werden 20 g Kaliumcanrenoat gelöst und 2 g Furosemid suspendiert. Durch Zugabe von Natriumhydroxydlösung unter Rühren wird das Furosemid gelöst und das pH der Lösung auf 10,8 eingestellt. Mit Wasser für Injektionszwecke wird auf 1 l aufgefüllt.

Die Lösung wird entkeimend filtriert, in Ampullen zu 10 ml abgefüllt und bei 120 °C 20 Minuten sterilisiert.

**Ansprüche** (für die Vertragsstaaten : BE, CH, DE, FR, GB, IT, LI, NL, SE)

1. Spritzfertige injizierbare wäßrige Lösung mit einem pH-Wert zwischen 10,2 und 11,2, dadurch gekennzeichnet, daß sie ein Gemisch der Alkalisalze von Canrenoinsäure (3-(3-Oxo-17β-hydroxy-4,6-androstadien-17α-yl)-propionsäure) und Furosemid (4-Chlor-N-(2-furyl-methyl)-5-sulfamoyl-anthranilsäure) ohne Pufferzusatz enthälten.

2. Lösung gemäß Anspruch 1, dadurch gekennzeichnet, daß sie Kaliumcanrenoat und das Natriumsalz von Furosemid enthält.

3. Verfahren zur Herstellung einer spritzfertigen injizierbaren wäßrigen Lösung mit einem pH-Wert zwischen 10,2 und 11,2, gemäß Anspruch 1, dadurch gekennzeichnet, daß man Canrenoinsäure oder Alkali-Canrenoat mit Furosemid in Wasser suspendiert, die Suspension mit wäßriger Alkalilösung auf einen pH-Wert von 10,2 bis 11,2 einstellt und so in eine klare Lösung überführt.

4. Verfahren gemäß Anspruch 3, dadurch gekennzeichnet, daß man Kaliumcanrenoat mit Furosemid in Wasser suspendiert und die Suspension mit Natriumhydroxidlösung auf pH 10,2 bis 11,2, vorzugsweise 10,6 bis 11,0, einstellt.

5. Verfahren gemäß Ansprüchen 3 und 4, dadurch gekennzeichnet, daß man auf einen pH-Wert von 10,8 einstellt.

**Ansprüche** (für den Vertragsstaat AT)

1. Verfahren zur Herstellung einer spritzfertigen injizierbaren wäßrigen Lösung mit einem pH-Wert zwischen 10,2 und 11,2, dadurch gekennzeichnet, daß man Canrenoinsäure (3-(3-Oxo-17β-hydroxy-4,6-androstadien-17α-yl)-propionsäure) oder Alkali-Canrenoat mit Furosemid (4-Chlor-N-(2-furyl-methyl)-5-sulfamoyl-anthranilsäure) in Wasser suspendiert, die Suspension mit wäßriger Alkalilösung auf einen pH-Wert von 10,2 bis 11,2 einstellt und so in eine klare Lösung überführt, welche keinen Pufferzusatz enthält.

2. Verfahren gemäß Anspruch 1, dadurch gekennzeichnet, daß man Kaliumcanrenoat mit Furosemid in Wasser suspendiert und die Suspension mit Natriumhydroxydlösung auf pH 10,2 bis 11,2, vorzugsweise 10,6 bis 11,0, einstellt.

3. Verfahren gemäß Ansprüchen 1 und 2, dadurch gekennzeichnet, daß man auf einen pH-Wert von 10,8 einstellt.

**Claims** (for the Contracting States : BE, CH, DE, FR, GB, IT, LI, NL, SE)

1. Ready for use, injectable, aqueous solution of a pH of from 10.2 to 11.2, characterized in that it contains a mixture of an alkali metal salt of canrenoic acid (3-(3-oxo-17β-hydroxy-4,6-androstadien-17α-yl)-propionic acid) and furosemide (4-chloro-N-(2-furylmethyl)-5-sulfamoyl-anthranilic acid) without addition of a buffer.

2. Solution as claimed in claim 1, containing potassium canrenoate and the sodium salt of furosemide.

3. Process for the manufacture of a ready for use, injectable, aqueous solution of a pH of from 10.2 to 11.2, as claimed in claim 1 which comprises suspending canrenoic acid or an alkali metal canrenoate and furosemide in water and adjusting the pH of the suspension to 10.2-11.2 by adding aqueous alkali solution, thereby transforming the suspension into a clear solution.

4. Process as claimed in claim 3, wherein potassium canrenoate and furosemide are suspended in water and the suspension is adjusted to pH 10.2 to 11.2, preferably 10.6 to 11.0, with sodium hydroxide solution.

5. Process as claimed in claims 3 and 4, wherein a pH of 10.8 is adjusted.

**Claims** (for the Contracting State AT)

1. Process for the manufacture of a ready for use, injectable, aqueous solution of a pH of from 10.2 to 11.2, which comprises suspending canrenoic acid (3-(3-oxo-17β-hydroxy-4,6-androstadien-17α-yl)-propionic acid) or an alkali metal canrenoate and furosemide (4-chloro-N-(2-furfurylmethyl)-5-sulfamoyl-anthranilic acid) in water and adjusting the pH of the suspension to 10.2-11.2 by adding aqueous alkali solution, thereby transforming the suspension into a clear solution.

2. Process as claimed in claim 1, wherein potassium canrenoate and furosemide are suspended in water and the suspension is adjusted to pH 10.2 to 11.2, preferably 10.6 to 11.0, with sodium hydroxid solution.

3. Process as claimed in claims 1 and 2 wherein a pH of 10.8 is adjusted.

**Revendications** (pour les Etats contractants ; BE, CH, DE, FR, GB, IT, LI, NL, SE)

1. Solution aqueuse directement injectable avec un pH compris entre 10,2 et 11,2, caractérisée en ce qu'elle contient un mélange de sels alcalins de l'acide canrénoïque (acide 3-(3-oxo-17β-hydroxy-4,6-androstadien-17α-yl)-propionique) et de furosémide (acide 4-chloro-N-(2-furyl-méthyl)-5-sulfamoyl-anthranilique) sans ajouter de tampon.

2. Solution selon la revendication 1, caractérisée en ce qu'elle contient du canrénoate de potassium et le sel de sodium de furosémide.

3. Procédé pour la préparation d'une solution aqueuse directement injectable avec un pH compris entre 10,2 et 11,2, selon la revendication 1, caractérisé en ce qu'on met l'acide canrénoïque ou un canrénoate alcalin avec le furosémide en suspension dans l'eau, on ajuste le pH de la suspension à une valeur de 10,2 à 11,2, avec une solution aqueuse d'alcali et on obtient ainsi une solution limpide.

4. Procédé selon la revendication 3, caractérisé en ce qu'on met le canrénoate de potassium avec le furosémide en suspension dans l'eau et on ajuste le pH de la suspension à une valeur de 10,2 à 11,2, de préférence de 10,6 à 11,0, avec une solution d'hydroxyde de sodium.

5. Procédé selon l'une des revendications 3 et 4, caractérisé en ce qu'on ajuste le pH à une valeur de 10,8.

**Revendications** (pour l'Etat contractant AT)

1. Procédé pour la préparation d'une solution aqueuse directement injectable ayant un pH compris entre 10,2 et 11,2, caractérisé en ce qu'on met l'acide canrénoïque (acide 3-(3-oxo-17β-hydroxy-4,6-androstadien-17α-yl)-propionique ou un canrénoate alcalin avec le furosémide (acide 4-chloro-N-(2-furyl-méthyl-5-sulfamoyl)-anthranilique) en suspension dans l'eau, on ajuste le pH de la suspension à une valeur de 10,2 à 11,2 avec une solution aqueuse d'alcali et on obtient ainsi une solution limpide qui ne contient pas de tampon ajouté.

2. Procédé selon la revendication 1, caractérisé en ce qu'on met le canrénoate de potassium avec le furosémide en suspension dans l'eau et on ajuste le pH de la suspension avec une solution d'hydroxyde de sodium à une valeur de 10,2 à 11,2, de préférence de 10,6 à 11,0.

3. Procédé selon l'une des revendications 1 et 2, caractérisé en ce qu'on ajuste le pH à une valeur de 10,8.